# EUROPEAN PATENT SPECIFICATION

(11) **EP 2 499 960 B1**
(45) Date of publication and mention of the grant of the patent: **22.04.2015**
(21) Application number: 11173756.5
(22) Date of filing: 13.07.2011
(51) Int. Cl.: A61B 3/00, A61B 3/107, A61B 3/11, A61B 3/113, G06F 3/01, G02B 27/00, G02B 27/01, A61B 3/024, A61B 3/14

(54) **Method for determining at least one parameter of two eyes by setting data rates and optical measuring device**
Verfahren zur Bestimmung von mindestens einem Parameter aus zwei Augen durch Einstellen der Datenraten und optische Messvorrichtung
Procédé pour déterminer au moins un paramètre de deux yeux en définissant des débits de données et dispositif de mesure optique

(30) Priority: 18.03.2011 EP 11158891
(43) Date of publication of application: 19.09.2012
(73) Proprietor: SensoMotoric Instruments Gesellschaft für innovative Sensorik mbH, 14513 Teltow (DE)
(72) Inventor: Nistico, Walter, 10119 Berlin (DE); Hoffmann, Jan, Dr., 14167 Berlin (DE); Schmidt, Eberhard, 14532 Kleinmachnow (DE)
(74) Representative: Hofstetter, Schurack & Partner

(56) References cited:
- EP-A1- 1 038 494
- EP-A1- 1 391 176
- WO-A1-2007/043954
- WO-A1-2010/118292
- US-A- 5 886 767
- US-A1- 2010 253 907

## Description

The invention relates to a method for determining at least one parameter of two eyes of a test person, the method comprising the steps of optically capturing of a first eye of the two eyes by means of a first capturing unit, of optically capturing the second eye of the two eyes by means of a second capturing unit, of transmitting first signals concerning the captured first eye from the first capturing unit to an analysis unit and transmitting second signals concerning the captured second eye from the second capturing unit to the analysis unit, and of determining the at least one parameter of each eye on the basis of the respective transmitted first and second signals in the analysis unit. The invention also relates to an optical measuring device for determining at least one parameter of two eyes of a test person.

It is known from the prior art to use head mounted eye tracker devices. US RE39,539 E discloses an apparatus for monitoring movement of a person's eye. The system includes a frame that is worn on a person's head, an array of emitters on the frame for directing light towards the person's eye, and an array of sensors on the frame for detecting light from the array of emitters. The sensors detect light that is reflected off respective portions of the eye or its eyelid, thereby producing output signals indicating when the reflective portion of the eye is covered by the eyelid. The system allows to monitor the persons level of drowsiness.

US 6,163,281 discloses a system and method for communication using movement of a person's eye, including an emitter for directing light towards an eye, a sensor for detecting emitted light from the emitter, and a processor coupled to the sensor for converting sequential light intensity signals received from the sensor to a stream of data, and/or for converting the signals into an understandable message.

US 2004/0196433 A1 discloses an eye tracking system for monitoring the movement of a user's eye comprising an eye camera and a scene camera for supplying to interlace electronics video data indicative of an image of the user's eye and an image of the scene observed by the user. In addition, the system incorporates a frame grabber for digitizing the video data and for separating the eye and scene data into two processing channels, and a spot location module for determining from the video data the location of a reference spot formed on the user's eye by illumination of the user's eye by a point source of light. The system further incorporates a pupil location module in order to determine the user's line of gaze.

WO 2010/83853 A1 discloses a gaze point detection system with one or more infrared signal sources to be placed in a test scene as reference points, at least one pair of eye glasses worn by a test subject, and a data processing and storage unit for calculating a gaze point of the person. The eye glasses comprise an image sensor adapted to detect IR signals from the at least one IR signal source and to generate an IR signal source tracking signal, an eye tracking unit adapted to determine the gaze direction of the test subject person and to generate an eye tracking signal, and a camera unit adapted to acquire a test scene picture.

WO 2004/066097 A2 discloses an eye tracking system for displaying a video screen pointer at a point of regard of a users gaze. The system comprises a camera focused on the user's eye, a support connected to the camera for fixing the relative position of the camera to the user's pupil, and a computer having a CPU and an eye tracking interface. By determining the center of the eye, a pointer on the video display screen can be displayed at the point of regard.

US 2010/0220291 A1 discloses an eye tracking system with a transparent lens, at least one light source, and a plurality of light detectors. The transparent lens is adapted for disposal adjacent an eye. At least one light source is disposed within the transparent lens and is configured to emit light towards the eye. The at least one light source is transparent to visible light. The plurality of light detectors is disposed within the transparent lens and is configured to receive light that is emitted from the at least one light source and is reflected off the eye. Each of the light detectors is transparent to visible light and is configured, upon receipt of light that is reflected off the eye, to supply an output signal.

Known head mounted eye trackers suffer from the disadvantage that large data quantities have to be processed in order to guarantee reliable eye tracking. Cameras monitoring the test person's eyes individually acquire and provide abundant data relating to characteristics concerning the eyes. This data needs to be transferred to an adequate processing unit quickly. Thus, significant bandwidth for the data transfer is required. Furthermore, the large data quantities have to get processed fast to achieve eye tracking in real time, i.e. with only little time delay. Consequently, expensive processing units are required, which suffer from high power consumption, too. In particular, mobile and small eye tracking devices may thus get heavy, bulky and expensive. In addition to a head mounted unit an external device comprising a processing unit may be necessary. State of the art devices, which are designed with leaner components, may suffer from not being able to keep pace with the abundant data stream. Loss of important data or significant time delay in the eye tracking functionality may be the result, thus compromising reliable determination of parameters characterizing a captured eye.

WO 2010/118292 A1 discloses an eye-tracking system and method including a display device, at least one image capture device and a processing device. The display device displays a user interface including one or more interface elements to a user. The image capture device detects a user's gaze location relative to the display device. The processing device electronically analyzes the location of user elements within the user interface relative to the user's gaze location and dynamically determines whether to initiate the display of a zoom window.

US 5,886,767 discloses improvements in the art of keratometry and, more particularly, to the use of television techniques to determine the shape of the corneal surface of an eye in essentially real-time. Paired television images of diffuse reflections from the cornea are produced by projecting an infra-red illuminated pattern onto the cornea. These diffuse reflections are compared by triangulation to define the corneal contour. The resultant data are processed by a conventional microcomputer to derive surface contour for display, the shape data so generated in a form for instant use.

An object of the present invention is to provide a method and an optical measuring device which allow for a more reliable determination of at least one parameter of two eyes of a test person.

This task according to the invention is solved by a method having the features according to patent claim 1, and an optical measuring device having the features according to patent claim 11. Advantageous embodiments of the invention are the subject-matter of the independent claims and the description.

The method according to the invention serves for determining at least one parameter of two eyes of a test person, the method comprising the following steps:
- optically capturing of a first eye of the two eyes by means of a first capturing unit;
- optically capturing the second eye of the two eyes by means of a second capturing unit;
- transmitting first signals concerning the captured first eye from the first capturing unit to an analysis unit and transmitting second signals concerning the captured second eye from the second capturing unit to the analysis unit;
- determining the at least one parameter of the two eyes on the basis of the transmitted first and second signals in the analysis unit; and
- setting a first data rate for the first signals and a second data rate for the second signals, wherein the first and the second data rate differ from each other, and wherein the transmitting of the first signals is effected at a first data rate and the transmitting of the second signals is effected at a second data rate.

As the first and second data rates differ from each other, one of the data rates is smaller than the other one. Thus, the overall bandwidth required for transmitting the first and second data is reduced compared to the state of the art. With the method it is, for example, possible to capture a first parameter characterizing the first eye and a second parameter, differing from the first parameter, characterizing the second eye. Data relating to the first parameter are then transmitted with the first data rate, while data concerning the second parameter are transmitted with the second data rate. Both parameters taken together then allow reliable determination of one or more parameters of interest characterizing the two eyes. In particular, first and second parameters do not need to be captured by both the first and second capturing unit. Capturing and transmission of redundant and superfluous information can be avoided. This way, data streams can be kept lean. Despite reduced data volume determining the at least one parameter of the two eyes can be performed more reliably.

The capturing unit may comprise at least one camera. It is possible that the capturing of the respective eye with the first and second capturing unit is effected at the same data rate. The acquired data may then be preprocessed such that data relating to the first capturing unit are transmitted to the analysis unit with the first data rate while the data relating to the second capturing unit are transmitted to the analysis unit with the differing second data rate. Alternatively, it may also be possible that already data acquisition by the first and second capturing units is effected at two differing data rates.

Advantageously, the method comprises the following steps:
- providing data concerning the respective captured eye in the first and/or the second capturing unit in dependency on the set first or second data rate;
- generating the first and/or second signals on the basis of the provided data.

The capturing or acquisition data rate of the first and second capturing units may thus differ. The first capturing unit may optically capture the first eye according to the set first data rate, while the second capturing unit may capture the second eye with the second data rate. This way, already in the step of data acquisition the amount of data is kept low. Only such data is acquired which is indeed necessary to determine the at least one parameter of the two eyes. Superfluous data are avoided right from the start. The overall data volume is kept low.

Advantageously, in the step of providing the data a data compression in dependency of the set first or second data rate is performed. In particular, this step can be performed after data is acquired by the first and second capturing unit but before the respective data is transmitted to the analysis unit. The step may comprise preprocessing of the data. Known state of the art data compression algorithms may be used. Data compression may be different for data provided by the first and second capturing units. For instance, data provided by the second capturing unit may undergo higher data compression than data provided by the first capturing unit. Different data compression algorithms may be performed in the first and second capturing units.

In one embodiment the method may comprise the following step: Setting a temporal capture resolution and/or a spatial capture resolution and/or a capturable image section, in particular a dynamic area of interest which follows the eye and can be adjusted with regard to its size and scan rate, of the first and/or the second capturing unit for the optical capturing of the respective eye in dependency on the set first or second data rate. In particular, a temporal capture resolution relates to a temporal data acquisition rate of the respective capturing unit. For instance, the first capturing unit may capture double the amount of signals during the same period of time as the second capturing unit. The spatial capture resolution may be defined by a pixelated resolution. If the spatial resolution of the first and second capturing unit is defined by the same pixel array with the same pixel size the spatial capture resolution of the first capturing unit may be four times as high as the spatial capture resolution of the second capturing unit if the second capturing unit performs a two-by-two binning. The capturable image section of the respective capturing unit may be a field of view of this capturing unit. This field of view may be dynamically adjusted to the gaze point of the respective captured eye. This way, first and second data acquisition rates can be flexibly adjusted. Depending on the respective situation an adequate first and/or second data rate can be chosen in an easy manner.

In one embodiment, the optical capturing of the first and second eye may occur with the same data rate but with a specific phase shift. While the first capturing unit is scanning the first eye, the second capturing unit may not scan the second eye and vice versa. The consecutive scanning intervals may be determined by a capturing rate, which may be the same for both capturing units. However, a time delay between scanning intervals of the first and second capturing unit may be introduced. If a parameter that is to be determined is virtually the same, irrespective of whether the first or second eye is observed, this redundancy together with the phase shift effectively results in an enhanced capturing rate. If, for example, the parameter that is to be determined is the pupil diameter, this diameter is usually the same for the first and second eye. The first and second eye may then be captured with a capturing rate of 30 Hz each, the two capturing sequences being shifted by half a period. The pupil diameter is then effectively sampled with 60 Hz rate. Varying the phase shift is thus an elegant way to set the desired data rate.

Advantageously, the method may comprise the following steps:
- optically capturing a field of view, which at least partly corresponds to a field of view capturable by the eyes of the test person, by means of a third capturing unit;
- transmitting third signals concerning the captured field of view from the third capturing unit to the analysis unit; and
- determining a correlation between the captured field of view and the at least one determined parameter on the basis of the first and third and/or second and third signals in the analysis unit.

In particular, the third capturing unit may be a scene camera. In a preferred embodiment depending on the first and third and/or second and third signals the captured field of view can be switched from a landscape into a portrait mode or vice versa. Consequently, the field of view captured by the third capturing unit may not be constant but may be adjusted depending, for example, on a point of regard of the two eyes, which may be determined on the basis of the first and third and/or second and third signals. Thus, only the field of view relevant in the respective situation is captured and unnecessary data is avoided. In other embodiments the third capturing unit may comprise several capturing units or sensors, e.g. a scene camera and an infrared camera.

Advantageously, the method comprises the following step: determining a third data rate for the third signals, wherein the transmitting of the third signals is effected at the third data rate, wherein the third data rate in particular is different from the first and/or second data rate. All three data rates may thus be chosen individually and in dependency of the respective capturing situation. If, for example, a high first data rate is required, the second and/or third data rate can be adjusted accordingly and respective lower values can be chosen for them.

In one embodiment the first data rate is set to be larger than the second data rate, and on the basis of the first and second signals a directional view and/or a visual focus of the test person is determined. It may be possible that solely by optically capturing the first eye with the first capturing unit a coarse direction of view and/or a coarse visual focus may be determinable in the analysis unit. Then the data acquired with the second capturing unit may allow a fine adjustment of the determined coarse direction of view and/or visual focus. Consequently, first and second data rates do not need to be equal but the second data rate can be chosen to be lower than the first data rate. This way, the directional view and/or visual focus can be determined very precisely despite reduced overall data rates.

Advantageously, on the basis of the first and the second signals and/or the first and the third signals and/or the second and the third signals in the analysis unit a parallax correction is performed. One of the group of the first, second or third signals may then serve as a primary data source for determining a point of regard. Another signal of the group of first, second and third signals may then serve as a corrective signal to perform the parallax correction with respect to the point of regard. Determining the point of regard may require a comparatively high amount of data and consequently a high data rate, while the data required for the parallax correction may be acquired and/or transmitted with a comparatively lower data rate. The overall data rate is kept low. Advantageously, the at least one captured parameter concerns an orientation and/or a position and/or an eyelid closure and/or a pupil diameter and/or a sclera characteristic and/or an iris characteristic and/or a characteristic of a blood vessel and/or a cornea characteristic of the at least one eye. In particular the at least one captured parameter may concern a cornea radius (anterior, posterior), an eyeball radius, a distance pupilcenter to cornea-center, a distance cornea-center to eyeball-center, a distance pupilcenter to limbus center, a cornea keratometric index of refraction, a cornea index of refraction, a vitreous humor index of refraction, a distance crystalline lens to eyeball-center and to cornea center and to corneal apex, a crystalline lens index of refraction, a visual axis orientation, an optical axis orientation, a pupil axis (achromatic axis) orientation, a line of sight orientation, an astigmatism degree (diopters) and orientation angle of flat and steep axis, an iris diameter, pupil diameters (pupil major and minor axes), pupil area), limbus major and minor axes, eye cyclo-torsion, eye intra-ocular distance, eye vergence, statistics over eye adduction/abduction and statistics over eye elevation/depression. The optical measuring device can then work as an eye tracking device.

An optical measuring device according to the invention serves for determining at least one parameter of two eyes of a test person and comprises a first capturing unit configured to optically capture a first eye of the two eyes, a second capturing unit configured to optically capture the second eye of the two eyes, an analysis unit configured to receive first signals concerning the captured first eye and transmitted by the first capturing unit and second signals concerning the captured second eye and transmitted by the second capturing unit, and on the basis of the transmitted first and second signals to determine the at least one parameter of the two eyes, and an assigning unit configured to set a first data rate for the first signals and a different second data rate for the second signals, so that the transmission of the first signals to the analysis unit is effected at the first data rate and the transmission of the second signals to the analysis unit is effected at the second data rate.

In particular, the analysis unit and the assigning unit may be comprised by a single processing unit and/or computer.

Advantageously, the optical measuring device may comprise a third capturing unit configured to capture a field of view which at least partly corresponds to a field of view which is capturable by the eyes of the test person and configured to transmit third signals concerning the captured field of view at a third data rate to the analysis unit, wherein the assigning unit is configured to set the third data rate. In particular, the third capturing unit may be a camera. While the first and second capturing units may be cameras observing the eyes of the test person, the third capturing unit may be a scene camera capturing a similar scene as seen by the test person. In particular, the first and second capturing units on the one hand and the third capturing unit on the other hand may be directed into opposite directions.

Advantageously, the assigning unit may be configured to set the ratio of the first to the second data rate and/or the ratio of the first to the third data rate and/or the ratio of the second to the third data rate to be such that it assumes a value in the range of 1:5000 to 5000:1. The wide range allows setting the respective data rates independently of each other. The overall data rate can be fine-tuned.

Advantageously, the assigning unit is configured to set the first and/or the second and/or the third data rate in dependency on each other and/or in dependency on predeterminable parameters, in particular a data transmission volume on a data line, and/or in dependency on a predeterminable measurement purpose of the optical measuring device. Consequently, one can make use of the full bandwidth provided by a data line of the optical measuring device without leaving any bandwidth unused or surpassing the maximum data volume that can be transmitted. The respective data rates can dynamically adjust to the respective situation.

Preferentially, the optical measuring device comprises at least one common data line configured to transmit the first and second and/or the first and third and/or the second and third signals. The bandwidth of a common data line is usually limited. By making the first, second and third data rates adjustable, one can make use of the full bandwidth provided. Compromising data by surpassing the allocatable bandwidth can be prevented.

Advantageously, the first and/or the second and/or the third capturing unit comprise at least one camera.

Further features of the invention derive from the claims, the figures, and the description of the figures. All features and feature combinations previously mentioned in the description as well as the features and feature combinations mentioned further along in the description of the figures and/or shown solely in the figures are not only usable in the combination indicated in each case, but also in different combinations or on their own.

The invention is now explained in more detail with reference to individual preferred embodiments and with reference to the attached drawings. These show in:
- Figure 1A: a front view of a spectacle device according to an embodiment of the invention;
- Figure 1B: a side view of the spectacle device of Figure 1A;
- Figure 1C: a top view of the spectacle device of Figure 1 A;
- Figure 1D: a perspective view of the spectacle device of Figure 1A;
- Figure 2: a rear view of a spectacle device;
- Figure 3: a schematic rear view of a spectacle device with an eye camera making use of a deflection element to direct its optical path onto the eye;
- Figure 4: a side view of a spectacle device schematically showing the orientation of an eye camera;
- Figure 5: a schematic view of individual electronic components comprised by a spectacle device;
- Figure 6A: a picture with a symbol indicating a large parallax error attained with an optical measuring device according to the prior art;
- Figure 6B: a picture showing a symbol indicating the lack of a parallax error with a spectacle device according to an embodiment of the invention;
- Figure 7: a parallax error model;
- Figure 8: a diagram comparing parallax errors of measuring devices according to the prior art and according to an embodiment of the invention;
- Figure 9A: a first field of view acquired by a scene camera;
- Figure 9B: a second field of view acquired by the scene camera;
- Figure 10A: a schematic side view of a spectacle device were the optical path of an eye camera extends in a straight line from the eye camera to an eye; and
- Figure 10B: a schematic side view of a spectacle device where the optical path of an eye camera extends from the eye camera via a mirror to the eye.

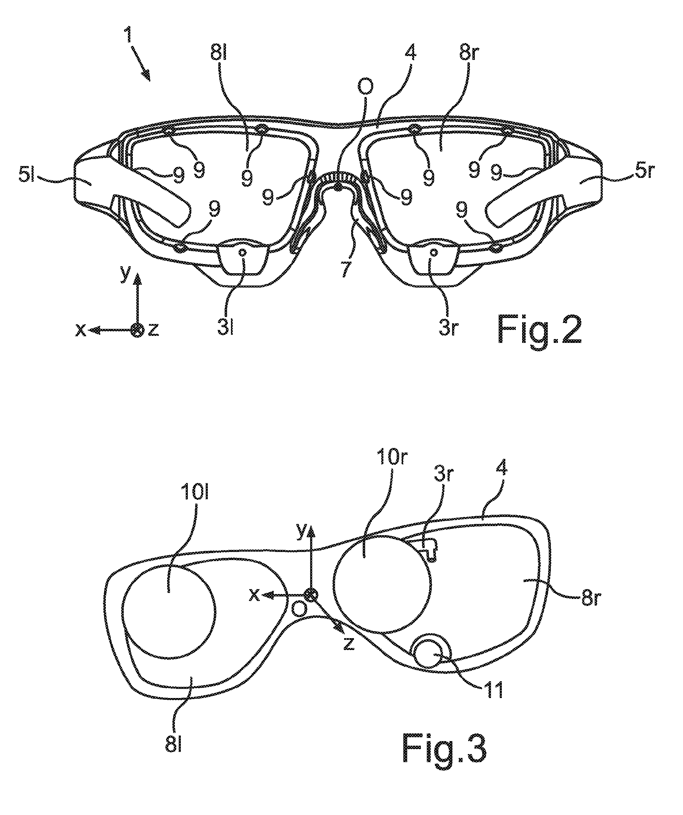

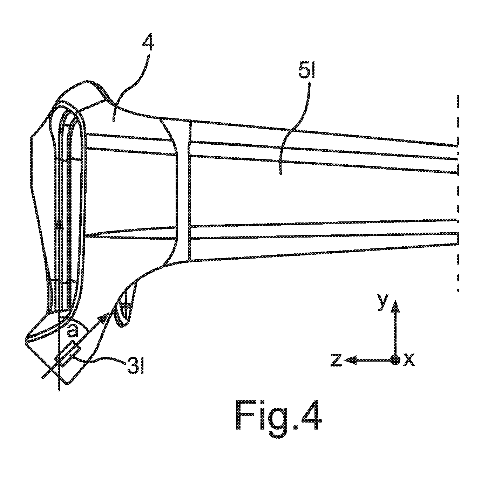

In the figures same elements or elements of the same function are equipped with the same reference signs. Figures 2, 3, and 4 show the same reference frame with a Cartesian coordinate system and perpendicular axes x, y and z.

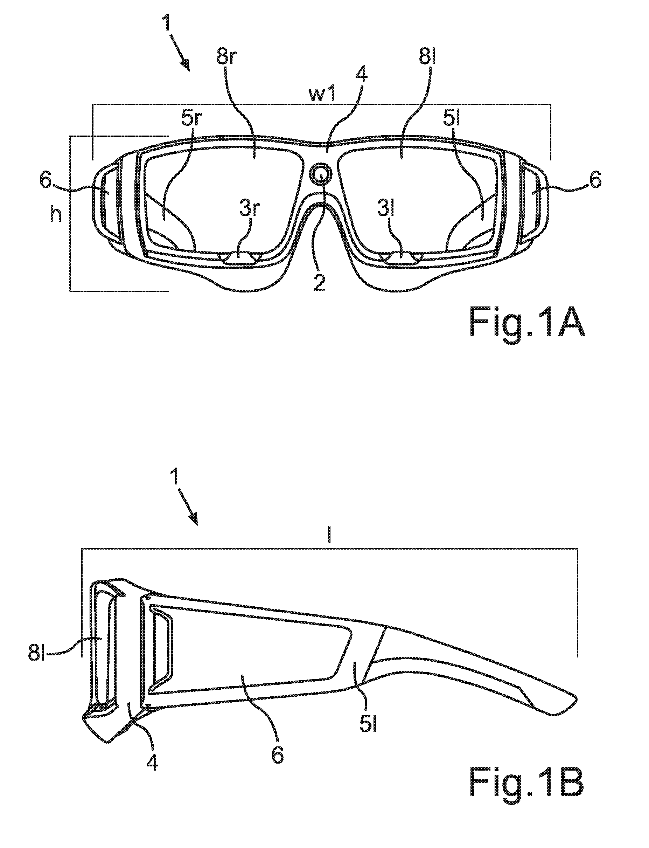

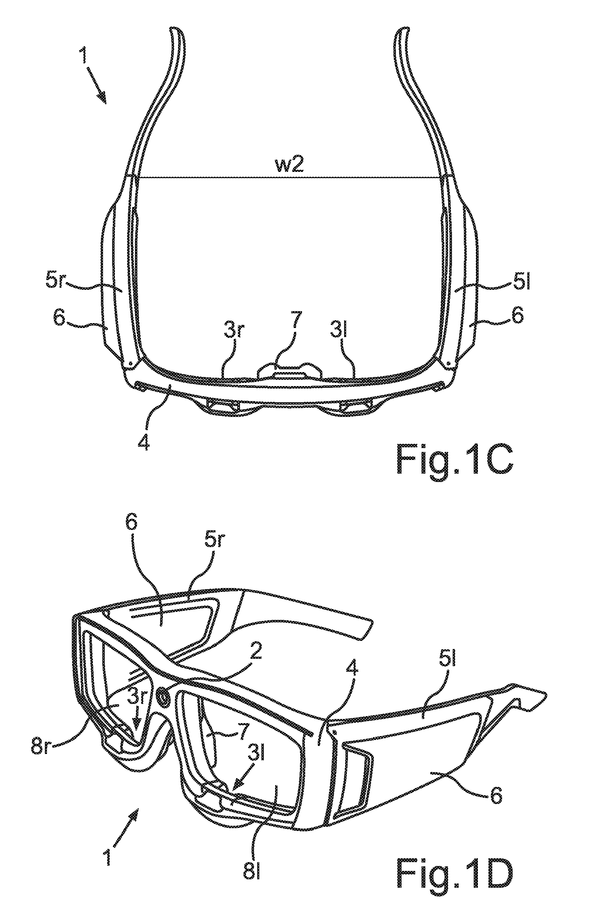

Figures 1A to 1D show an optical measuring device which has the form of a spectacle device 1 or eye tracking device, respectively. The spectacle device 1 is designed such that a person can wear it on its head just like a normal pair of glasses. It comprises a frame 4 with two side bars 5l and 5r which support the spectacle device 1 on the ears of the person who is wearing it. Furthermore, the spectacle device 1 is held in place on the head by a nose support 7. The mainframe has a specific width w1 and height h. Its length I depends on the length of the sidebars 5l and 5r. As can be seen in Figure 1 C the sidebars 5l and 5r are hinged to the front part of the frame 4 such that the distance w2 between the side bars 5l and 5r can be enlarged or reduced (see dashed sidebar configuration for sidebar 5l in Figure 1C).

Alternatively, the optical measuring device may not be designed in form of a regular pair of eye glasses, but may be designed such that it resembles a helmet, forming a frame, with a face shield, forming a frame insert.

Above the nose support 7 in the frame 4 a scene camera 2 is installed. It can either be attached to or integrated into the frame 4. With the scene camera 2 virtually a similar field of view can be captured as seen by a test person when wearing the spectacle device 1. In the lower part of the frame 4 the spectacle device 1 contains two eye cameras 3l and 3r. When the spectacle device 1 is worn by a person the person's eyes can be captured by the eye cameras 3l and 3r, which are integrated into the frame 4 at a suitable angle. Eye cameras 3l and 3r are designed to observe the person's left eye and right eye, respectively, i.e. capture characteristics of the person's eyes.

The frame 4 contains two openings which are filled with eye glass lenses 8l and 8r thus forming frame inserts. The pictures acquired by the scene camera 2 and the eye cameras 3l and 3r lead to signals which are processed in one or several pre-processing units 6 integrated into the sidebars 5l and 5r.

Figure 2 shows an inside view of the spectacle device 1. Along the rim of the frame part enclosing the eye glass lenses 8l and 8r several Light Emitting Diods (LEDs) 9 are located in a ring arrangement. When the spectacle device 1 is worn by a person, those LEDs 9 can illuminate the eyes of the test person in a defined way. The LEDs 9 will cause reflections on the eyes of the test person (cornea reflections) for all possible gaze angles. Those reflections can be detected by the eye cameras 3l and 3r and can be used for eye tracking.

The LEDs 9 can be switched on an off individually, in groups or all together following a specific time pattern, strobe characteristic or spatial variation. The on-off-switching-frequency of different LEDs 9 or groups of LEDs 9 may vary. Certain groups of LEDs 9 may get switched on exactly when other groups of LEDs 9 get switched off. A specific spatial and temporal correlation pattern may be implemented with regard to the switching and thus illumination characteristics. This way a reflection pattern can be created on the eyes that can be recognized easily by the eye cameras 3.

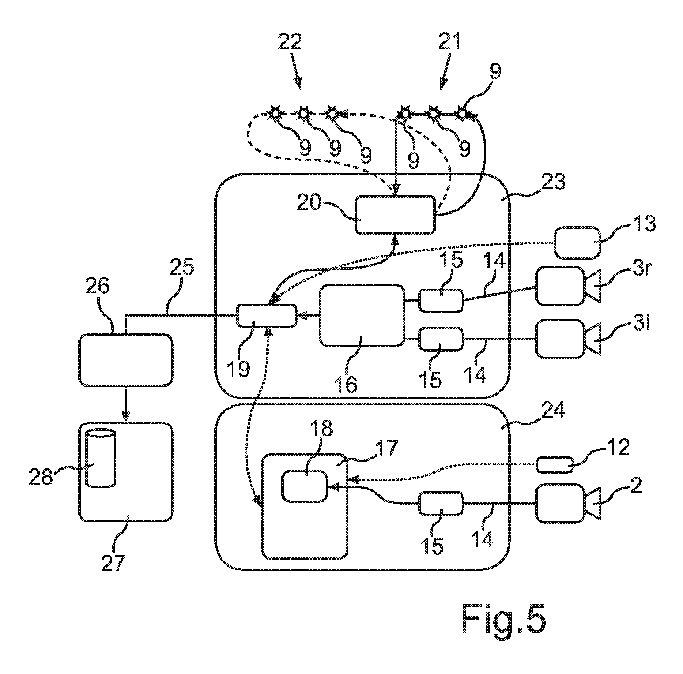

The overall setup with the most important electronic components is shown in Figure 5. The eye cameras 3l and 3r are connected to specific camera electronics 15 by 100 mm long cables 14. In particular, the cameras 3l and 3r comprise only basic electronic components while their major electronic components are located within the camera electronics 15. This way, the primarily "optical part" of the cameras 3l and 3r can be located remote to the primarily "electronic part" within the camera electronics 15. Both parts can then be connected by flex-PCB cables 14. This way, the optical sensor and the basic electronic components within the cameras 3l and 3r form a very small and highly compact entity while bulkier electronic components within the electronics 15 can be placed on more spacious integrated circuit boards elsewhere. The electronics 15 are connected to a pre-processing unit 16 which can process the signals from the eye cameras 3l and 3r. The pre-processing unit 16 can be identical to the pre-processing unit 6 located in the sidebars 5l and 5r of the spectacle device 1. The pre-processing unit 16 is connected to a USB-hub 19. The LEDs 9 installed in the frame 4 form a first and a second IR LED chain 21 and 22 arranged in a ring configuration around the eye glass lenses 8l and 8r. The IR LED chains 21 and 22 are connected to an IR LED constant current source 20, which is also connected to the USB-hub 19. The USB-hub 19 additionally serves as a power source for the IR LED constant current source 20. The LEDs 9 of the IR LED chains 21 and 22 can be switched on an off individually. To achieve this, they may be connected to the IR LED constant current source 20 in a parallel network with individual electrical switches for each LED 9 being implemented.

The USB-hub 19 is connected via a serial interface or USB cable 25 to a pre-processing unit 26. The signals pre-processed in the pre-processing unit 26 are finally analyzed in a personal computer 27, which contains a recorder device 28. An additional aux-/sync-port 13 forming an interface on the spectacle device 1 can also be connected to the USB-hub 19. The aux-/sync-port 13 can serve as interface for synchronization with other electronic devices or for triggering parallel data acquisitions. The electronics 15, pre-processing unit 16, USB-hub 19 and IR LED constant current source 20 are located on a common printed circuit board PCB 23.

In analogy to this setup the scene camera 2 is also connected to electronics 15 via a 100 mm cable 14. In this case the electronics 15 are located on a second printed circuit board PCB 24, which also contains a pre-processing unit 17. The pre-processing unit 17 can be based on electronics according to the DaVinci digital signal processor (DSP). It contains an MPEG encoder 18 for encoding the signals received from the electronics 15. A microphone 12 may also be connected to the pre-processing unit 17. The pre-processing unit 17 located on the PCB 24 is connected to the USB-hub 19. This way, processing signals acquired by the scene camera 2 are finally analyzed in the personal computer 27.

The pre-processing units 6, 16, 17 and 26 may be able to compress at least one of the three image streams generated by the two eye cameras 3l and 3r and the scene camera 2. Here, different alternatives are possible. A pre-processing unit may compress only the image stream of one camera while each camera has its own pre-processing unit. Alternatively, a single pre-processing unit may compress the image streams of all cameras. Furthermore, the pre-processing units may be configurable via a system interface and corresponding software to manage the bandwidth by adjustment of resolution, region of interest, frame rate and compression parameters. The pre-processing units may be designed to trigger synchronously the camera's image acquisition. They may provide time stamps for each acquired image which can be used to synchronise several or all camera data streams offline.

The pre-processing units may either be located on integrated circuit boards of the cameras or on a separate integrated circuit board that is located at or on a head mount (e. g. in the side bar 5l or 5r of the spectacle device 1) or in a separate housing that is worn by the test person 31, e.g. on a belt.

The spectacle device 1 may also comprise an auxiliary interface which allows to acquire data in real time from external sensors. Such sensors may be biometric sensors (including but not limited to EEG, ECG, etc.) or attitude sensors (including but not limited to accelerometers, magnetometers, gyroscopes, etc.). It is then possible to synchronise the data stream of the external sensors with the data streams acquired from the cameras 2, 3l and 3r. Furthermore, an external clock or trigger signal can be provided that can be used by the external sensors to synchronise themselves with the system. The bandwidth of data acquired from the interface can be reduced or compressed by means of on-board processing resources integrated in the system in its dedicated recording unit 28.

The eye cameras 3l and 3r can either be suited for visible or near infrared light. They are located symmetrically with respect to a vertical centre line that divides the user's face into two halves. The eye cameras 3l and 3r may be positioned in front and below the eyes 10l and 10r respectively, for example in or at the lower rim of a pair of eye glass lenses 8l and 8r, pointing at the eyes 101 and 10r in an angle of 30 ° to 50°and being mounted in the frame 4 in an angle a of 30°to 50°. In the embodiment the eye cameras 3l and 3r are sensitive in the near infrared.

In the embodiment the eye cameras 3l and 3r are sensitive in the near infrared. They have a resolution of 640*480 and are read out with a 60 Hz frequency.

The scene camera 2 can be located on a vertical centre line that divides the user's face into two halves in or at the nose bridge of the frame 4. Alternatively, it may also be located at, in or close to the rim of a helmet, cap or headband. The scene camera 2 may have HD (high definition) and/or adjustable resolution. It can either be mounted in landscape or portrait orientation. Furthermore, it can be mounted such that its orientation can be changed from landscape to portrait orientation (camera roll) and also the direction the camera is pointing in (camera pan and tilt).

Instead of a single scene camera 2, the spectacle device 1 can also comprise a pair of scene cameras, where each scene camera can be oriented either in portrait mode or in landscape mode. Furthermore, each scene camera can be oriented independently of the respective second scene camera. Alternatively, both scene cameras 2 may have fixed orientations, which may or may not differ from each other.

Furthermore a prism or lens can be mounted in front of the scene camera 2 to create a different positioning of the field of view of the scene camera 2 with respect to the glasses, especially a more downward oriented field of view for near range reading applications.

Six LEDs 9 are located around each eyeglass lens 8. They emit in the infrared wavelength range (typically above 750 nm and below 1000 nm) at a central wavelength of 850 nm. They are driven by 50 mA current provided by the IR LED constant current source 20.

Instead of direct illumination of the eyes with the LEDs 9 also an implementation with a light guide can be envisaged. One or several segments of light guides (e.g. fiber optics) may be used. The illumination of the eyes may be implemented with focusing optics (structured illumination). Instead of the LEDs 9 suitable diffractive optics or lasers may be used to generate a pattern of coherent light for illuminating the eyes. The light source can be used together with an optical element in order to create a pattern of reflections on the eyes 10l and 10r (e.g. with focusing optics or diffractive optics). The illumination source may either emit visible or near infrared light. The illumination source may be positioned in or on the frame 4, in particular in a circle-like arrangement around the eye glass lenses 8l and 8r. Alternatively, the illumination source may be located on the rim or frame of a head mounted display. It may specifically be designed to create a pattern of reflections on the eye surfaces of the test person 31.

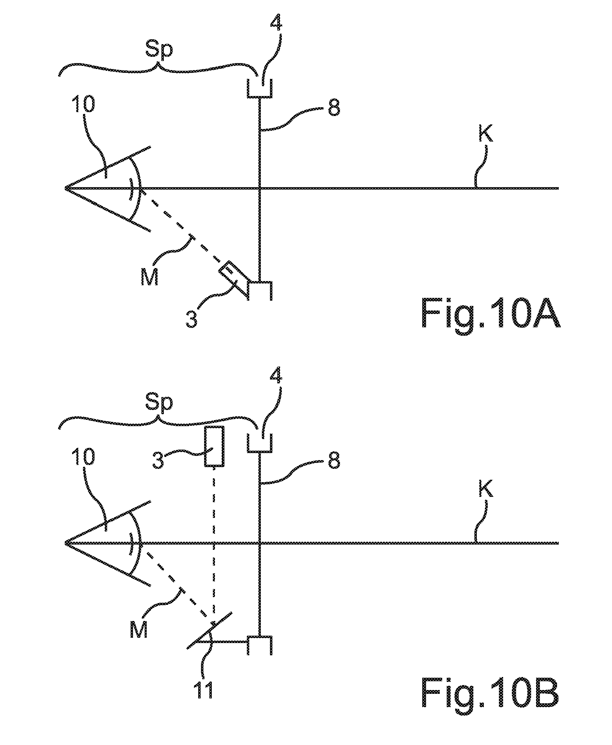

When the spectacle device 1 shown in Figure 2 is worn by a test person the situation shown in Figure 10A in a simplified way is realized. The eye camera 3 is arranged in such a way on the frame 4 that with the spectacle device 1 fixed to the head of a test person the optical path M capturing at least one parameter of the eye 10 extends in a straight line from the eye camera 3 to the eye 10.

Figures 3 and 10B show a different configuration of the spectacle device 1. The spectacle device 1 comprises a mirror 11, forming an optical deflection element attached to the frame 4, the mirror 11 and the eye camera 3 being arranged in such a way on the frame 4 that with the spectacle device 1 fixed to the head of the test person the optical path M for capturing at least one parameter of the eye 10 extends from the eye camera 3 via the mirror 11 to the eye 10. The three dimensional representation of Figure 3 shows the spectacle device 1 from a rear or inside view. In the figure, reflections of the left and right eye 10l and 10r, respectively, show in the eyeglass lenses 8l and 8r. The coordinate system is a Cartesian one with the z-axis being directed into the plane of projection.

Thus, the eye cameras 3l and 3r may be mounted in front of and above the eyes 10l and 10r with an optical guide or mirror 11 located in front and below the eyes 10l and 10r, for example in or at the lower rim of a pair of eye glass lenses 8l and 8r in order to acquire an image of each eye 10l and 10r from a forward and low perspective and to make that image visible to the eye cameras 10l and 10r. The optical guide or mirror 11 can either be a (flat) mirror, a spherical mirror, a dome, a custom lens, a holographic image guide, etc. The mirror 11 can be reflecting only a specific range of wavelength and be transparent to others.

The mirror 11 can either be a flat mirror or a spherical mirror. The advantage of a spherical mirror is that it magnifies the field of view of the eye camera 3 beyond the field of view achievable with a flat mirror. The configuration of Figure 3 furthermore allows to place the optical system very close to the eye 10 (set direction) thus improving ergonomics and aesthetics. The test person's own field of view is hardly obstructed. The mirror 11 can be a so-called hot mirror, i.e. the mirror 11 is transparent in the visible wavelength range while having a higher reflectivity in the infrared wavelength range. It can be very thin and hollow (so-called dome) thus, minimizing the distortion due to refraction. It can be made out of a material showing a very low index of refraction (IOR).

In both cases (Figures 10A and 10B) the eye camera 3 is arranged in such a way that the optical path M for the capturing of at least one parameter of the eye 10 excludes the frame insert, i.e., the eye glass lens 8. Furthermore, the eye glass lens 8 is arranged in such a way that the optical axis K of the eye 10 and the optical path M as single jointly used optical element comprise the eye 10. Furthermore, the optical path M entirely runs within a space Sp which extends on the side of the eye glass lens 8 facing the eye 10.

The embodiments shown in Figures 2 and 3 and Figures 10A and 10B, respectively, both reduce eye occlusion due to the upper eye-lid.

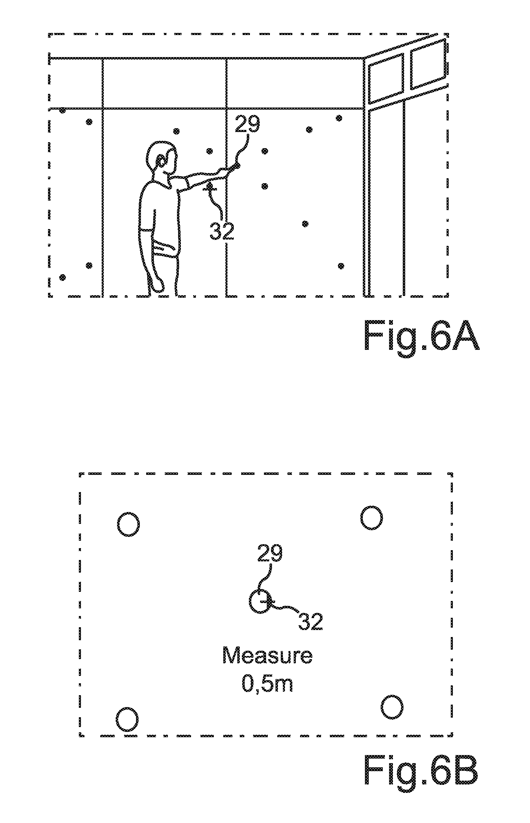

Figures 6A to 8 illustrate the reduction of parallax errors in the spectacle device 1 compared to the prior art. As can be seen in Figure 6A the position of an object 29 the test person actually focuses its eyes on and the point of regard 32 determined by the spectacle device 1 usually do not coincide very well when using spectacle devices 1 as known from the prior art. This effect is usually the more pronounced the closer the test person is located to the object 29 that is to be focused. However, with the spectacle device 1 according to an embodiment of the invention the coincidence between the determined point of regard 32 and the actual object 29 is very good, even for measuring distances as low as 0.5 m (see Figure 6B). This is achieved by minimizing the distance between the eye ball center and the camera focal point.

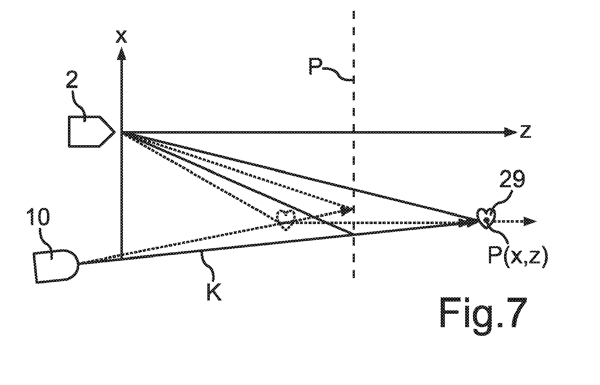

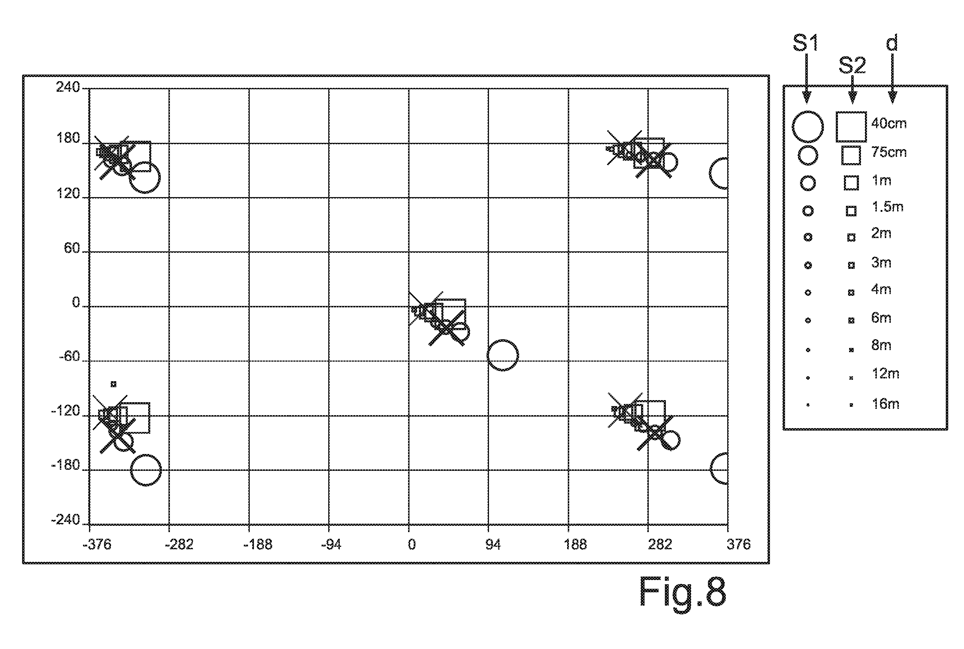

The situation is again illustrated in Figure 7. As eye 10 and scene camera 2 are located at slightly different positions the difference in their respective viewing angles for focussing the object 29 becomes the more pronounced the closer the object 29 is located to the eye 10 and scene camera 2, respectively (i.e. larger distortions for smaller z-values). The spectacle device 1 may get calibrated in the situation shown in Figure 6B. The object 29 then lies in the calibration plain P and by calibrating the spectacle device 1 one can make sure that the determined point of regard 32 indeed falls onto the actual object 29. Calibration is typically performed on a plane at some distance from the test subject. It relates measured gaze direction (angles) to pixels in the scene video frame. This calculation gives valid results only for points that lie in that calibration plane. For points that do not lie on that plane, a systematic error (parallax) is introduced. When the distance of the spectacle device from the object 29 is increased the difference between the distance to the calibration plain P and the actual distance to the object 29 causes the pronounced deviations. With the spectacle device 1 according to an embodiment of the invention these deviations or parallax errors (indicated by symbols S2, circles, in Figure 8) for all distances d are considerably smaller than with devices according to the prior art (symbols S1, rectangles). Thin-lined crosses relate to the group of symbols S2, while bold crosses relate to the group of symbols S1. The crosses correspond to the point of regard 32 used for calibration purposes.

The parallax error is mathematically modelled as a function of the position of the scene camera 2 with respect to the eye position. The gaze estimation error due to parallax is minimized by placing the scene camera 2 as close as possible to the eye 10, according to the results shown by the mathematical simulation. The parallax error can be further corrected by estimating the distance to the point of regard by using vergence from binocular tracking and by estimating the position of the eyes with respect to the eye tracking device.

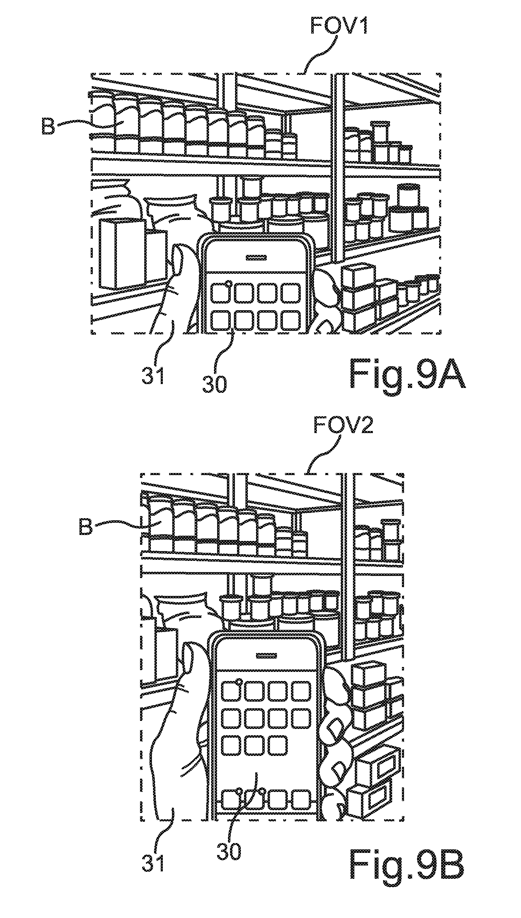

To achieve even better results the field of view of the scene camera 2 can be optimized. The scene camera 2 with standard optics has a field of view that does not cover the full physiological gaze range (horizontal field of view of standard optics: 40 ° to 50°; typical physiological gaze range: 60°). In an embodiment the field of view of the scene camera 2 can thus be optimized depending on the respective application. One such field of view optimization method is illustrated in Figure 9A and 9B. A user wearing the spectacle device 1 is at the same time observing a background B and his mobile phone 30. According to Figure 9A the field of view FOV1 mainly covers the background B. When the test person 31 looks down onto its mobile phone 30 the change in gaze direction is automatically determined by the eye cameras 3l and 3r and the scene camera's 2 field of view is automatically adjusted by switching from landscape to portrait orientation (field of view FOV2). This can be achieved by a z-axis 90° mechanical roll of the scene camera 2 or by the use of an optical prism in front of the scene camera 2. Also the use of two scene cameras with different tilt or roll angles is possible. Alternatively, also an optical beam splitter may be used in front of the scene camera 2.

In summary, the spectacle device 1 forms a head-mounted eye tracking system which consists of three cameras: two eye cameras 3l and 3r and at least one scene camera 2. The three cameras 3l, 3r and 2 can have a manageable bandwidth, for example by adjustable frame rates or resolutions. One or several pre-processing units 6, 16, 17 and 26 may exist that perform variable compression of the video streams received from the cameras 2, 3l and 3r. The level of compression of the video streams may be the same for the eye cameras 3l and 3r and the scene camera 2, or the video streams may be separately compressed for the eye cameras 3l and 3r and the scene camera 2. The frame rate for eye camera 3l may correspond to full speed acquisition, the one of eye camera 3r may correspond to 1/10 speed acquisition and the one for the scene camera 2 may correspond to 1/2 speed acquisition. Instead of adjusting the frame rates of the different cameras, alternatively the acquisition rates may be chosen to be the same, while data processing is performed differently for each camera. Data provided by one camera may be compressed more than data provided by another camera, although both cameras acquire the same amount of data. One may also combine different compression rates with different acquisition rates. It is also possible to omit, for example, every second acquired image when transferring the data and thus reduce the amount of data to be sent to the CPU by half. The signals of the cameras 2, 3l and 3r may be transferred to a CPU in the PC 27 via a wired or wireless interface (see Figure 5). Auxiliary interfaces for other data sources and methods for synchronisation with these data sources may be implemented in the spectacle device 1.

The spectacle device 1 can come as a system comprising several exchangeable pieces. The spectacle device 1 can have an exchangeable set of nose pieces or nose supports 7 for faces with small or large noses. This way, the spectacle device 1 can be worn over vision correction glasses without a problem. Furthermore, the spectacle device 1 has a holding mechanism for exchangeable glasses that can have different levels of light transmittance (e g. clear glasses or sun glasses) for a certain range of wavelengths. Additionally or alternatively the exchangeable glasses can have a near infrared optical filter to match the wavelength of the illumination source and block some or all light from the outside of same and similar wavelengths from reaching the eye surface to improve signal to noise on the eye surface. The spectacle device 1 has rims and a nose bridge that serve as a mount or housing for the eye cameras 3l and 3r and the scene camera 2. The eye cameras 3l and 3r are mounted in such a way that their field of view extends behind the exchangeable glasses 8l and 8r.

With the spectacle device 1 it is possible to do eye tracking, occulometrics, biometrics and position and motion measurements in order to measure and classify as fully as possible human behaviour in a free range movement setup. A head mounted eye tracking device is realised which is calibration-free and provides an astigmatism estimation. The eye-tracking functionality has zero set-up time. No adjustments are necessary. A test person 31 can just put the spectacle device 1 on and start using it. It has a very large gaze-tracking range covering the physiological range of human eye movement (80° horizontal, 60° vertical). It is very robust and has a high accuracy in gaze mapping. Astigmatism is compensated for, parallax is minimized, pupil axis shift is compensated and the device is calibration free or can be calibrated using a one-point calibration feature. Furthermore, it is designed to work irrespective of ethnic group (Caucasian, Asian, African, etc.), gender and age. The field of view of the scene camera 2 is optimized. By the use of optical, inertial or magnetic sensors a head tracking functionality can be implemented. The spectacle device furthermore offers biometric features, such as measuring the pupil diameter and offering interfacing and synchronisation options with EEG, ECG, etc. Finally, it can be integrated with a head mounted display. It is possible to project a virtual image onto a subject's eye of a portable computer screen. Furthermore, the possibility is offered to interact with "objects" in the virtual image using eye movement (gaze, blinks).

Head tracking functionality can be realized by the use of three axis gyroscopes, three axis accelerometers and/or three axis magnetometers with optional sensor fusion for six dimensional head tracking.

In summary, the spectacle device 1 offers a very specific optical and electronic architecture. With respect to the electronic architecture three or more high resolution cameras with allocateable bandwidth are incorporated in the device 1. Separate processing channels for eye cameras 3l and 3r and the scene camera 2 are envisaged. The optical architecture is characterized by exchangeable glasses with various properties. The optical path of the eye cameras 3l and 3r extends behind the glasses or eye glass lenses 8l and 8r respectively. Furthermore, a set of LEDs 9 allows for highly variable illumination of the eyes 10l and 10r. For instance, the illumination geometry around the eye can be controlled. The specific LED subsets can be controlled with regard to strobe effect and sequencing. Finally, eye illumination can be achieved by point, line or two-dimensional light sources.

### REFERENCE SIGNS:

- 1: spectacle device
- 2: scene camera
- 3, 3l, 3r: eye camera
- 4: frame
- 5l, 5r: side bar
- 6: pre-processing unit
- 7: nose support
- 8, 8l, 8r: eyeglass lens
- 9: LED
- 10, 10l, 10r: eye
- 11: mirror
- 12: microphone
- 13: aux-/sync-port
- 14: cable
- 15: electronics
- 16: pre-processing unit
- 17: pre-processing unit
- 18: MPEG encoder
- 19: USB hub
- 20: IR LED constant current source
- 21, 22: IR LED chain
- 23, 24: PCB
- 25: USB 2.0 cable
- 26: pre-processing unit
- 27: PC
- 28: recorder
- 29: object
- 30: mobile phone
- 31: test person
- 32: point of regard
- w1, w2: width
- h: height
- l: length
- a: tilt angle
- K: optical axis
- M: optical path
- O: origin of system of reference
- P: calibration plane
- Sp: space
- d: distance
- S1, S2: symbols
- B: background
- FOV1, FOV2: field of view
- x, y, z: axis

## Claims

1. A method for determining at least one parameter of two eyes (10l, 10r) of a test person (31), the method comprising the following steps:
- optically capturing of a first eye (10l; 10r) of the two eyes (10l, 10r) by means of a first capturing unit (3l; 3r);
- optically capturing the second eye (10r; 10l) of the two eyes (10l, 10r) by means of a second capturing unit (3r; 3l);
- transmitting first signals concerning the captured first eye (10l; 10r) from the first capturing unit (3l; 3r) to an analysis unit (27) and transmitting second signals concerning the captured second eye (10r; 10l) from the second capturing unit (3r; 3l) to the analysis unit (27);
- determining the at least one parameter of each eye (10l, 10r) on the basis of the respective transmitted first and second signals in the analysis unit (27),
**characterized by** the following step:
- setting a first data rate for the first signals and a second data rate for the second signals, wherein the first and the second data rate differ from each other, and wherein transmitting of the first signals is effected at the first data rate and transmitting of the second signals is effected at the second data rate.

2. The method according to claim 1,
**characterized by** the following steps:
- providing data concerning the respective captured eye (10l, 10r) in the first and/or the second capturing unit (3l, 3r) in dependency on the set first or second data rate;
- generating the first and/or second signals on the basis of the provided data.

3. The method according to claim 2,
**characterized in that**
in the step of providing the data a data compression in dependency of the set first or second data rate is performed.

4. The method according to one of the preceding claims,
**characterized by** the following step:
setting a temporal capture resolution and/or a spatial capture resolution and/or a capturable image section, in particular a dynamic area of interest, which follows the eye (10l, 10r) and can be adjusted with regard to its size and scan rate, of the first and/or the second capturing unit (3l, 3r) for the optical capturing of the respective eye (10l, 10r) in dependency on the set first or second data rate.

5. The method according to one of the preceding claims,
**characterized by** the following steps:
- optically capturing a field of view (FOV1, FOV2), which at least partly corresponds to a field of view capturable by the eyes (10l, 10r) of the test person (31), by means of a third capturing unit (2);
- transmitting third signals concerning the captured field of view (FOV1, FOV2) from the third capturing unit (2) to the analysis unit (27);
- determining a correlation between the captured field of view (FOV1, FOV2) and the at least one determined parameter on the basis of the first and third and/or second and third signals in the analysis unit (27).

6. The method according to claim 5,
**characterized by** the following step:
- determining a third data rate for the third signals, wherein the transmitting of the third signals is effected at the third data rate, wherein the third data rate in particular is different from the first and/or second data rate.

7. The method according to claim 5 or 6,
**characterized in that**
the first data rate is set to be larger than the second data rate, and on the basis of the first and second signals a direction of view (K) and/or a visual focus (32) of the test person (31) is determined.

8. The method according to one of the preceding claims,
**characterized in that**
on the basis of the first and the second signals and/or the first and the third signals and/or the second and the third signals in the analysis unit (27) a parallax correction is performed.

9. The method according to one of the preceding claims,
**characterized in that**
the transmitting of the first and/or second and/or third signals is effected via a common data line (25).

10. The method according to one of the preceding claims,
**characterized in that**
the at least one captured parameter concerns an orientation and/or a position and/or an eyelid closure and/or a pupil diameter and/or a sclera characteristic and/or an iris characteristic and/or a characteristic of a blood vessel and/or a cornea characteristic of the at least one eye (10l, 10r).

11. An optical measuring device (1) for determining at least one parameter of two eyes (10l, 10r) of a test person (31), the optical measuring device (1) comprising:
- a first capturing unit (3l; 3r) configured to optically capture a first eye (10l; 10r) of the two eyes (10l, 10r);
- a second capturing unit (3r; 3l) configured to optically capture the second eye (10r; 10l) of the two eyes (10l, 10r);
- an analysis unit (27) configured to receive first signals concerning the captured first eye (10l; 10r) and being transmitted by the first capturing unit (3l;
3r), second signals concerning the captured second eye (10r; 101) and being transmitted by the second capturing unit (3r; 31), and, on the basis of the respective transmitted first and second signals, determine the at least one parameter of each eye (101, 10r),
**characterized by**
an assigning unit (27) configured to set a first data rate for the first signals and a different second data rate for the second signals, so that the transmission of the first signals from the first capturing unit (3l; 3r) to the analysis unit (27) is effected at the first data rate and the transmission of the second signals from the second capturing unit (3r; 3l) to the analysis unit (27) is effected at the second data rate.

12. The optical measuring device (1) according to claim 11,
**characterized by**
a third capturing unit (2) configured to capture a field of view (FOV1, FOV2) which at least partly corresponds to a field of view which is capturable by the eyes (10l, 10r) of the test person (31), and configured to transmit third signals concerning the captured field of view (FOV1, FOV2) at a third data rate to the analysis unit (27), wherein the assigning unit (27) is configured to set the third data rate.

13. The optical measuring device (1) according to claim 11 or 12,
**characterized in that**
the assigning unit (27) is configured to set the ratio of the first to the second data rate and/or the ratio of the first to the third data rate and/or the ratio of the second to the third data rate to be such that it assumes a value in the range of 1/5000 to 5000/1.

14. The optical measuring device (1) according to one of claims 11 to 13,
**characterized in that**
the assigning unit (27) is configured to set the first and/or the second and/or the third data rate in dependency on each other and/or in dependency on pre-determinable parameters, in particular a data transmission volume on a data line (25), and/or in dependency on a pre-determinable measurement purpose of the optical measuring device (1).

15. The optical measuring device (1) according to one of claims 11 to 14,
**characterized by**
at least one common data line (25) configured to transmit the first and second and/or the first and third and/or the second and third signals.

## Patentansprüche

1. Verfahren zum Bestimmen zumindest eines Parameters von zwei Augen (10l, 10r) eines Probanden (31) mit den folgenden Schritten:
- optisches Erfassen eines ersten Auges (10l; 10r) der zwei Augen (10l, 10r) mittels einer ersten Erfassungseinheit (3l; 3r);
- optisches Erfassen des zweiten Auges (10r; 10l) der zwei Augen (10l, 10r) mittels einer zweiten Erfassungseinheit (3r; 3l);
- Übertragen von ersten Signalen betreffend das erfasste erste Auge (10l; 10r) von der ersten Erfassungseinheit (3l; 3r) an eine Auswerteeinheit (27) und Übertragen von zweiten Signalen betreffend das erfasste zweite Auge (10r; 10l) von der zweiten Erfassungseinheit (3r; 3l) an die Auswerteeinheit (27);
- Bestimmen des zumindest einen Parameters jedes Auges (10l, 10r) anhand der jeweiligen übertragenen ersten und zweiten Signale in der Auswerteeinheit (27), **gekennzeichnet durch** den Schritt:
- Festlegen einer ersten Datenrate für die ersten Signale und einer zweiten Datenrate für die zweiten Signale, wobei die erste und zweite Datenrate voneinander verschieden sind, und wobei das Übertragen der ersten Signale mit der ersten Datenrate und das Übertragen der zweiten Signale mit der zweiten Datenrate erfolgt.

2. Verfahren nach Anspruch 1,
**gekennzeichnet durch** die folgenden Schritte:
- Bereitstellen von Daten, welche das jeweils erfasste Auge (10l, 10r) betreffen, in der ersten und/oder zweiten Erfassungseinheit (3l, 3r) in Abhängigkeit von der festgelegten ersten bzw. zweiten Datenrate;
- Erzeugen der ersten und/oder zweiten Signale anhand der bereitgestellten Daten.

3. Verfahren nach Anspruch 2,
**dadurch gekennzeichnet, dass**
im Schritt des Bereitstellens der Daten eine Datenkompression in Abhängigkeit von der festgelegten ersten bzw. zweiten Datenrate ausgeführt wird.

4. Verfahren nach einem der vorhergehenden Ansprüche,
**gekennzeichnet durch** den folgenden Schritt:
Festlegen einer zeitlichen Erfassungsauflösung und/oder einer räumlichen Erfassungsauflösung und/oder eines erfassbaren Bildausschnitts, insbesondere eines dem Auge (10l, 10r) folgenden und in seiner Größe und Abtastrate festlegbaren, dynamischen Area of Interest, der ersten und/oder zweiten Erfassungseinheit (3l, 3r) für das optische Erfassen des jeweiligen Auges (10l, 10r) in Abhängigkeit von der festgelegten ersten bzw. zweiten Datenrate.

5. Verfahren nach einem der vorhergehenden Ansprüche,
**gekennzeichnet durch** die folgenden Schritte:
- optisches Erfassen eines Sichtfeldes (FOV1, FOV2), welches zumindest teilweise einem Sichtfeld entspricht, welches von den Augen (10l, 10r) des Probanden (31) erfassbar ist, mittels einer dritten Erfassungseinheit (2);
- Übertragen von dritten Signalen betreffend das erfasste Sichtfeld (FOV1, FOV2) von der dritten Erfassungseinheit (2) an die Auswerteeinheit (27);
- Bestimmen einer Korrelation zwischen dem erfassten Sichtfeld (FOV1, FOV2) und dem zumindest einen bestimmten Parameter anhand der ersten und dritten und/oder zweiten und dritten Signale in der Auswerteeinheit (27).

6. Verfahren nach Anspruch 5,
**gekennzeichnet durch** den folgenden Schritt:
- Festlegen einer dritten Datenrate für die dritten Signale, wobei das Übertragen der dritten Signale mit der dritten Datenrate erfolgt, wobei die dritte Datenrate insbesondere verschieden von der ersten und/oder zweiten Datenrate ist.

7. Verfahren nach Anspruch 5 oder 6,
**dadurch gekennzeichnet, dass**
die erste Datenrate größer als die zweite Datenrate festgelegt wird, und anhand der ersten und zweiten Signale eine Blickrichtung (K) und/oder ein Blickpunkt (32) des Probanden (31) bestimmt wird.

8. Verfahren nach einem der vorhergehenden Ansprüche,
**dadurch gekennzeichnet, dass**
anhand der ersten und zweiten Signale und/oder ersten und dritten Signale und/oder zweiten und dritten Signale in der Auswerteeinheit (27) eine Parallaxenkorrektur durchgeführt wird.

9. Verfahren nach einem der vorhergehenden Ansprüche,
**dadurch gekennzeichnet, dass**
das Übertragen der ersten und/oder zweiten und/oder dritten Signale über eine gemeinsame Datenleitung (25) erfolgt.

10. Verfahren nach einem der vorhergehenden Ansprüche,
**dadurch gekennzeichnet, dass**
der zumindest eine erfasste Parameter eine Orientierung und/oder eine Position und/oder einen Lidschluss und/oder einen Pupillendurchmesser und/oder eine Sklera-Charakteristik und/oder eine Iris-Charakteristik und/oder eine Charakteristik eines Blutgefäßes und/oder eine Hornhaut-Charakteristik des zumindest einen Auges (10l, 10r) betrifft.

11. Optische Messvorrichtung (1) zur Bestimmung zumindest eines Parameters von zwei Augen (10l, 10r) eines Probanden (31) mit:
- einer ersten Erfassungseinheit (3r; 3l), welche dazu ausgebildet ist, ein erstes Auge (10l; 10r) der zwei Augen (10l, 10r) optisch zu erfassen;
- einer zweiten Erfassungseinheit (3l; 3r), welche dazu ausgebildet ist, das zweite Auge (10r; 10l) der zwei Augen (10l, 10r) optisch zu erfassen;
- einer Auswerteeinheit (27), welche dazu ausgebildet ist, von der ersten Erfassungseinheit (3l; 3r) übertragene erste Signale betreffend das erfasste erste Auge (10l; 10r) und von der zweiten Erfassungseinheit (3r; 3l) übertragene zweite Signale betreffend das erfasste zweite Auge (10r; 10l) zu empfangen, und anhand der jeweiligen übertragenen ersten und zweiten Signale den zumindest einen Parameter eines jeden Auges (10l, 10r) zu bestimmen,
**gekennzeichnet durch**
eine Zuordnungseinheit (27), welche dazu ausgebildet ist, eine erste Datenrate für die ersten Signale und eine hiervon verschiedene zweite Datenrate für die zweiten Signale festzulegen, so dass die Übertragung der ersten Signale von der ersten Erfassungseinheit (3l; 3r) an die Auswerteeinheit (27) mit der ersten Datenrate und die Übertragung der zweiten Signale von der zweiten Erfassungseinheit (3r; 3l) an die Auswerteeinheit (27) mit der zweiten Datenrate erfolgt.

12. Optische Messvorrichtung (1) nach Anspruch 11,
**gekennzeichnet durch**
eine dritte Erfassungseinheit (2), welche dazu ausgelegt ist, ein Sichtfeld (FOV1, FOV2) zu erfassen, welches zumindest teilweise einem Sichtfeld entspricht, welches von den Augen (10l, 10r) des Probanden (31) erfassbar ist, und dazu ausgelegt ist, dritte Signale betreffend das erfasste Sichtfeld (FOV1, FOV2) mit einer dritten Datenrate an die Auswerteeinheit (27) zu übertragen, wobei die Zuordnungseinheit (27) dazu ausgebildet ist, die dritte Datenrate festzulegen.

13. Optische Messvorrichtung (1) nach Anspruch 11 oder 12,
**dadurch gekennzeichnet, dass**
die Zuordnungseinheit (27) dazu ausgebildet ist, das Verhältnis der ersten zur zweiten Datenrate und/oder das Verhältnis der ersten zur dritten Datenrate und/oder das Verhältnis der zweiten zur dritten Datenrate so festzulegen, dass dieses einen Wert im Bereich von 1/5000 bis 5000/1 annimmt.

14. Optische Messvorrichtung (1) nach einem der Ansprüche 11 bis 13,
**dadurch gekennzeichnet, dass**
die Zuordnungseinheit (27) dazu ausgebildet ist, die erste und/oder zweite und/oder dritte Datenrate in Abhängigkeit voneinander und/oder in Abhängigkeit von vorgebbaren Parametern, insbesondere einem Datenübertragungsvolumen auf einer Datenleitung (25), und/oder in Abhängigkeit eines vorgebbaren Messzwecks der optischen Messvorrichtung (1) festzulegen.

15. Optische Messvorrichtung (1) nach einem der Ansprüche 11 bis 14,
**gekennzeichnet durch**
zumindest eine gemeinsame Datenleitung (25), welche dazu ausgebildet ist, die ersten und zweiten und/oder die ersten und dritten und/oder die zweiten und dritten Signale zu übertragen.

## Revendications

1. Procédé, destiné à déterminer au moins un paramètre de deux yeux (10l, 10r) d'un individu soumis à un essai (31), le procédé comprenant les étapes suivantes consistant à :
- capturer optiquement un premier oeil (10l, 10r) sur les deux yeux (10l, 10r) au moyen d'une première unité de capture (3l, 3r) ;
- capturer optiquement le second oeil (10r, 10l) sur les deux yeux (10l, 10r) au moyen d'une deuxième unité de capture (3r, 3l) ;
- transmettre des premiers signaux concernant le premier oeil capturé (10l, 10r) depuis la première unité de capture (3l, 3r) à une unité d'analyse (27) et transmettre des deuxièmes signaux concernant le second oeil capturé (10r, 10l) depuis la deuxième unité de capture (3r, 3l) à l'unité d'analyse (27) ;
- déterminer le au moins un paramètre de chaque oeil (10l, 10r) sur la base des premiers et deuxièmes signaux transmis respectifs dans l'unité d'analyse (27),
**caractérisé par** l'étape suivante consistant à
- définir un premier débit de données pour les premiers signaux et un deuxième débit de données pour les deuxièmes signaux, dans lequel le premier et le deuxième débit de données diffèrent l'un de l'autre et dans lequel la transmission des premiers signaux est effectuée au premier débit de données et la transmission des second signaux est effectuées au deuxième débit de données.

2. Procédé selon la revendication 1,
**caractérisé par** les étapes suivantes consistant à
- fournir des données concernant l'oeil capturé respectif (10l, 10r) dans la première et / ou la deuxième unité de capture (3l, 3r) en fonction du premier ou deuxième débit de données défini ;
- générer les premiers et / ou deuxièmes signaux sur la base des données fournies.

3. Procédé selon la revendication 2,
**caractérisé en ce que**
dans l'étape, consistant à fournir les données, une compression de données est effectuée en fonction du premier ou deuxième débit de données défini.

4. Procédé selon l'une des revendications précédentes, **caractérisé par** l'étape suivante consistant à
- définir une résolution de capture temporelle et / ou une résolution de capture spatiale et / ou une partie d'image capturable, en particulier un centre dynamique d'intérêt qui suit l'oeil (10l, 10r) et peut être ajusté par rapport à sa taille et à sa fréquence de balayage, de la première et / ou la deuxième unité de capture (3l, 3r) pour la capture optique de l'oeil respectif (10l, 10r) en fonction du premier ou deuxième débit de données défini.

5. Procédé selon l'une des revendications précédentes, **caractérisé par** les étapes suivantes consistant à
- capturer optiquement un champ de vision (FOV1, FOV2), qui correspond au moins partiellement à un champ de vision capturable par les yeux (10l, 10r) de l'individu soumis à l'essai (31), au moyen d'une troisième unité de capture (2) ;
- transmettre des troisièmes signaux concernant le champ de vision capturé (FOV1, FOV2) depuis la troisième unité de capture (2) à l'unité d'analyse (27) ;
- déterminer une corrélation entre le champ de vision capturé (FOV1, FOV2) et le au moins un paramètre déterminé sur la base des premiers et troisièmes et / ou deuxièmes et troisièmes signaux dans l'unité d'analyse (27).

6. Procédé selon la revendication 5,
**caractérisé par** l'étape suivante consistant à
- déterminer un troisième débit de données pour les troisièmes signaux, dans lequel la transmission des troisièmes signaux est effectuée au troisième débit de données, dans lequel le troisième débit de données en particulier est différent du premier et / ou deuxième débit de données.

7. Procédé selon la revendication 5 ou 6,
**caractérisé en ce que**
le premier débit de données est défini comme étant supérieur au deuxième débit de données et une direction du regard (K) et / ou une acuité visuelle (32) de l'individu soumis à l'essai (31) est déterminée sur la base des premiers et deuxièmes signaux.

8. Procédé selon l'une des revendications précédentes,
**caractérisé en ce que**,
une correction de la parallaxe est effectuée sur la base des premiers et des deuxièmes signaux et / ou des premiers et des troisièmes signaux et / ou des deuxièmes et des troisièmes signaux dans l'unité d'analyse (27).

9. Procédé selon l'une des revendications précédentes,
**caractérisé en ce que**
la transmission des premiers et / ou deuxièmes et / ou troisièmes signaux est effectuée par l'intermédiaire d'une ligne de données commune (25).

10. Procédé selon l'une des revendications précédentes,
**caractérisé en ce que**
le au moins un paramètre capturé concerne une orientation et / ou une position et / ou un clignement des paupières et / ou un diamètre de pupille et / ou une caractéristique sclérotique et / ou une caractéristique de l'iris et / ou une caractéristique d'un vaisseau sanguin et / ou une caractéristique de la cornée du au moins un oeil (10l, 10r).

11. Dispositif de mesure optique (1), destiné à déterminer au moins un paramètre de deux yeux (10l, 10r) d'un individu soumis à un essai (31), le dispositif de mesure optique (1) comprenant :
- une première unité de capture (3l, 3r), configurée pour capturer optiquement un premier oeil (10l, 10r) sur les deux yeux (10l, 10r) ;
- une deuxième unité de capture (3l, 3r), configurée pour capturer optiquement le second oeil (10r, 10l) sur les deux yeux (10l, 10r) ;
- une unité d'analyse (27), configurée pour recevoir des premiers signaux concernant le premier oeil capturé (10l, 10r) et émis par la première unité de capture (3l, 3r), des deuxièmes signaux concernant le second oeil capturé (10r, 10l) et émis par la deuxième unité de capture (3r, 3l) et déterminer le au moins un paramètre de chaque oeil (10l, 10r) sur la base des premiers et deuxièmes signaux émis respectifs,
**caractérisé par**
une unité d'attribution (27), configurée pour définir un premier débit de données pour les premiers signaux et un deuxième débit de données différent pour les deuxièmes signaux, de sorte que la transmission des premiers signaux par la première unité de capture (3l, 3r) à l'unité d'analyse (27) est effectuée au premier débit de données et la transmission des deuxièmes signaux par la deuxième unité de capture (3r, 3l) à l'unité d'analyse (27) est effectuée au deuxième débit de données.

12. Dispositif de mesure optique (1) selon la revendication 11,
**caractérisé par**
une troisième unité de capture (2), configurée pour capturé un champ de vision (FOV1, FOV2) qui correspond au moins partiellement à un champ de vision qui est capturable par les yeux (10l, 10r) de l'individu soumis à l'essai (31) et configurée pour transmettre des troisièmes signaux concernant le champ de vision capturé (FOV1, FOV2) à un troisième débit de données à l'unité d'analyse (27), dans lequel l'unité d'attribution (27) est configurée pour définir le troisième débit de données.

13. Dispositif de mesure optique (1) selon la revendication 11 ou 12,
**caractérisé en ce que**
l'unité d'attribution (27) est configurée pour définir le rapport du premier au deuxième débit de données et / ou le rapport du premier au troisième débit de données et / ou le rapport du deuxième au troisième débit de données, de telle sorte qu'il suppose une valeur dans la plage de 1/5000 à 5000/1.

14. Dispositif de mesure optique (1) selon l'une des revendications 11 à 13,
**caractérisé en ce que**
l'unité d'attribution (27) est configurée pour définir le premier et / ou le deuxième et / ou le troisième débit de données en fonction l'un de l'autre et / ou en fonction de paramètres pouvant être prédéterminés, en particulier un volume d'émission de données sur une ligne de données (25) et / ou en fonction d'un objectif de mesure pouvant être prédéterminé du dispositif de mesure optique (1).

15. Dispositif de mesure optique (1) selon l'une des revendications 11 à 14,
**caractérisé par**
au moins une ligne de données commune (25), configurée pour émettre les premiers et deuxièmes et / ou les premiers et troisièmes et / ou les deuxièmes et troisièmes signaux.
